# EUROPÄISCHE PATENTSCHRIFT

(11) **EP 1 781 612 B1**
(45) Veröffentlichungstag und Bekanntmachung des Hinweises auf die Patenterteilung: **11.04.2012**
(21) Anmeldenummer: 05759650.4
(22) Anmeldetag: 12.07.2005
(51) Int. Cl.: C07D 211/32, A61K 31/445, A61P 25/30

(54) **VERKÜRZTES VERFAHREN ZUR HERSTELLUNG VON L-LOBELIN**
SHORTENED METHOD FOR THE PRODUCTION OF L-LOBELINE
PROCEDE RACCOURCI POUR LA PRODUCTION DE L-LOBELINE

(30) Priorität: 17.07.2004 DE 102004034682
(43) Veröffentlichungstag der Anmeldung: 09.05.2007
(73) Patentinhaber: Boehringer Ingelheim Pharma GmbH & Co. KG, 55216 Ingelheim (DE)
(72) Erfinder: KLINGLER, Franz-Dietrich, 64347 Griesheim (DE); SOBOTTA, Rainer, 55218 Ingelheim (DE)
(74) Vertreter: Hammann, Heinz
(86) Internationale Anmeldenummer: PCT/EP2005/007533
(87) Internationale Veröffentlichungsnummer: WO 2006/008029

(56) Entgegenhaltungen:
- WO-A-00/43345
- GB-A- 314 532
- FELPIN ET AL: "A highly stereoselective asymmetric synthesis of L-Lobeline and -Sedamine" JOC, Bd. 67, 2002, Seiten 9192-9199, XP002347128 in der Anmeldung erwähnt

## Beschreibung

Die vorliegende Erfindung betrifft ein verkürztes Verfahren zur Herstellung von L-Lobelin mittels Rhodium-katalysierter asymmetrischer Hydrierung im industriellen Maßstab.

### TECHNOLOGISCHER HINTERGRUND DER ERFINDUNG

Lobelin gehört zu den Lobelia Alkaloiden der Lobelia inflata (Indianer-Tabak, Brechkraut, Asthmagras) und ist in den östlichen und mittleren Staaten der USA und Kanadas heimisch. Die Pflanze enthält ca. 0,3% Alkaloide. Bei den Lobelia Alkaloiden handelt es sich um 2,6-disubstituierte Piperidin-Derivate. Unter den etwa 20 Lobelia Alkaloiden ist Lobelin das Hauptalkaloid. Lobelin wirkt parenteral (3-10 mg) verabreicht atemstimulierend und wurde früher als Atem-Analeptikum bei Asthma, Kollaps u. Narkoseunfällen gegeben. Oral genommen wird es rasch abgebaut und ist deshalb unwirksam. Da Lobelin die Wirkung des Nicotins verstärkt und dadurch Brechreiz und Ekelgefühl hervorruft, wird es klinisch in Depotform als Mittel zur Raucherentwöhnung entwickelt (Drug News (25.3) 1996, 6).

### STAND DER TECHNIK

Die Synthese von L-Lobelin ist im Stand der Technik bekannt. Bisherige Verfahren weisen jedoch eine verhältnismäßig hohe Anzahl von Reaktionsschritten auf. So wird zum Beispiel in einer jüngeren Publikation zu diesem Thema eine stereoselektive Synthese von Lobelin vorgeschlagen, die ausgehend von Benzaldehyd 17 Reaktionsschritte bis zum Erhalt des Produktes benötigt (Felpin et al., J. Org. Chem (2002) 67, 9192).

Aufgabe der vorliegenden Erfindung ist es also, ein verkürztes Verfahren zur Herstellung von L-Lobelin bereit zu stellen.

Überraschenderweise wurde nun gefunden, dass man Lobelin der Formel I im technischen Maßstab in guten Ausbeuten und guter optischer Reinheit erhalten kann, wenn man ein entsprechendes Diketon II einer asymmetrischen Hydrierung in Gegenwart von Rhodium und einem chiralen, zweizähnigen Phosphinliganden als Katalysatorsystem unterwirft.

### DETAILLIERTE BESCHREIBUNG DER ERFINDUNG

Die vorliegende Erfindung betrifft ein Verfahren zur Herstellung von L-Lobelin, der Formel I, 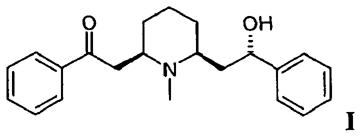 oder ein Säureadditionssalz davon, ausgehend von Lobelanin der Formel **II**, 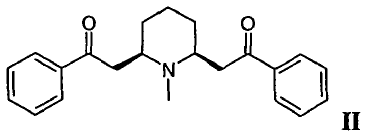 oder einem Säureadditionssalz davon, dadurch gekennzeichnet, dass man dieses einer asymmetrischen Hydrierung in Gegenwart eines Katalysatorsystems bestehend aus Rhodium und (2R,4R)-4-(Dicyclohexylphosphino)-2-(diphenylphosphino-methyl)-N-methyl-aminocarbonyl-pyrrolidin, gegebenenfalls einem inerten Verdünnungsmittels und einer schwachen Base unterwirft.

Das Ausgangsprodukt der Formel II kann man durch einfaches Umsetzen von 3-Oxo-3-phenylpropionsäure, 1,5-Pentandion und Methylamin-Hydrochlorid in Aceton und in Gegenwart eines Citratpuffers erhalten. 3-Oxo-3-phenylpropionsäure ist durch Verseifung des entsprechenden Ethylesters erhältlich.

Als inerte Verdünnungsmittel können sowohl protische Lösungsmittel - wie z.B. Alkohole und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁₋₈-Alkohole eingesetzt. Besonders bevorzugt werden niedere Alkohole wie Methanol, Ethanol, n-Propanol und iso-Propanol oder deren Mischungen eingesetzt. Besonders bevorzugt wird als Reaktionsmedium Methanol verwendet, wobei das Methanol oder die anderen Alkohole oder Lösungsmittel gegebenenfalls Wasser enthalten kann (können). Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon. Bevorzugt werden Lösungsmittel eingesetzt, die wenig zur Brennbarkeit neigen.

Bevorzugt wird das obige Verfahren, worin man die Hydrierung in Gegenwart von weniger als einem Äquivalent einer schwachen Base ausgewählt aus der Gruppe der tertiären Amine, Alkalimetallhydrogencarbonate und Alkalimetallcarbonate, durchgeführt wird.

Als organische Basen eignen sich tertiäre Amine, insbesondere tertiäre Alkylamine, tertiäre Alkyl-Arylamine oder Pyridine. Bevorzugt werden Trialkylamin mit verzweigten oder unverzweigten C₁₋₆-Alkylresten eingesetzt. Als ganz besonders bevorzugt haben sich beispielsweise Triethylamin oder Diisopropylethylamin bewährt. Gegebenenfalls kann die Reaktion auch in Gegenwart von basischen Polymeren mit z.B. tertiären Aminofunktionen durchgeführt werden.

Bevorzugt wird das obige Verfahren, worin die asymmetrische Hydrierung in einem Temperaturbereich von 0°C bis 100°C, bevorzugt 20-80°C, besonders bevorzugt 40-60°C durchgeführt wird.

Ebenfalls bevorzugt wird das obige Verfahren worin die asymmetrische Hydrierung unter einem Druck von 1 bis 40 bar, bevorzugt 10 bis 30 bar, besonders bevorzugt 15 bis 25 bar, durchgeführt wird.

In einer bevorzugten Ausführungsform der Erfindung wird das Verfahren unter Schutzgasatmosphäre durchgeführt, bevorzugt sind hierbei Stickstoff- oder Argonatmosphäre, oder Mischungen davon.

Am meisten bevorzugt wird das obige Verfahren, worin man eine Verbindung der Formel **II** oder ein Säureadditionssalz davon zum Rhodiumkatalysator bei der asymmetrischen Hydrierung in einem Mol-Verhältnis von 500:1 bis 100000:1, besonders bevorzugt 750:1 bis 20000:1, einsetzt.

Demzufolge ist am meisten bevorzugt ein Verfahren zur Herstellung von L-Lobelin, worin folgende Schritte durchgeführt werden:
- Lösen von Lobelanin in Methanol,
- Zugabe von Triethylamin in Methanol,
- Zugabe der Katalysator-Lösung, bevorzugt im Molverhältnis von 500:1 bis 100000:1, besonders bevorzugt 750:1 bis 20000:1,
- Beaufschlagung der Reaktionsmischung mit Wasserstoff ,
- Erhitzen der Reaktionsmischung auf 20-80°C, bevorzugt 40-60°C, besonders bevorzugt 47-53°C und Einstellen des Wasserstoffdruck auf 10-30 bar, bevorzugt 15-25 bar, besonders bevorzugt 20 bar einstellen,
- Nach Beendigung der Reaktion, Abdestillieren des Methanols unter reduziertem Druck.

Die Aufarbeitung der Reaktion kann in üblicher Weise erfolgen, zum Beispiel, indem man den Katalysator gegebenenfalls deaktiviert und abtrennt, das Lösungsmittel entfernt aus dem Rückstand reines Endprodukt durch Kristallisation, Destillation, Extraktion oder Chromatographie isoliert.

Bevorzugt ist ein Verfahren, worin zur Isolierung des Produktes folgende Schritte durchgeführt werden:
(i) Verteilung der bei der asymmetrischen Hydrierung erhaltenen Reaktionsmischung zwischen Wasser und einem organischen Lösungsmittel,
(ii) Einstellen eines pH-Wertes der wässrigen Phase im sauren Bereich,
(iii) Abtrennen der wässrigen Phase,
(iv) gegebenenfalls Wiederholung der Schritte (i) bis (iii)
(v) Einstellen des pH-Wertes der wässrigen Phase im basischen Bereich;
(vi) Verteilung der Reaktionsmischung zwischen Wasser und einem organischen Lösungsmittel,
(vii) gegebenenfalls Wiederholung der Schritte (v) bis (vi)
(viii) Abtrennen der gebildeten organischen Phase und Konzentrierung.
(ix) Kristallisieren des Produktes

Besonders bevorzugt ist das obige Verfahren mit anschließender Isolierung nach den Schritten (i) bis (ix), worin das organische Lösungsmittel Toluol ist.

Am meisten bevorzugt ist das obige Verfahren mit anschließender Isolierung nach den Schritten (i) bis (ix), worin das Produkt in Schritt (ix) durch Zugabe eines -C₁₋₈-Alkohols, bevorzugt *iso*-Propanol, kristallisiert wird.

### VERWENDETE BEGRIFFE UND DEFINITIONEN

Unter dem Begriff "C₁₋₈-Alkohol" werden verzweigte und unverzweigte Alkohole mit 1 bis 8 Kohlenstoffatomen verstanden und einer oder zwei Hydroxygruppen. Dem entsprechend werden unter dem Begriff "C₁₋₄Alkohole" verzweigte und unverzweigte Alkylgruppen mit 1 bis 4 Kohlenstoffatomen und einer oder zwei Hydroxygruppen verstanden. Bevorzugt sind Alkohole mit 1 bis 4 Kohlenstoffatome. Beispielsweise werden hierfür genannt: Methanol, Ethanol, *n*-Propanol, *iso*-Propanol, *n*-Butanol, *iso*-Butanol, *sec*-Butanol, *tert-*Butanol, *n*-Pentanol, *iso*-Pentanol, *neo*-Pentanol oder Hexanol. Gegebenenfalls werden für vorstehend genannte Moleküle auch die Abkürzungen MeOH, EtOH, *n*-PrOH, *i*-PrOH, *n-*BuOH, *i*-BuOH, *t*-BuOH, etc. verwendet. Sofern nicht anders beschrieben, umfassen die Definitionen Propanol, Butanol, Pentanol und Hexanol alle denkbaren isomeren Formen der jeweiligen Reste. So umfasst beispielsweise Propanol *n*-Propanol und *iso*-Propanol, Butanol umfasst *iso*-Butanol, *sec*-Butanol und *tert*-Butanol etc.

Gegebenenfalls können die Verbindungen der Formel **I** und **II** in ihre Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Oxalsäure, Phosphorsäure, Salzsäure, Schwefelsäure, Weinsäure oder Zitronensäure in Betracht. Ferner können Mischungen der vorgenannten Säuren eingesetzt werden.

Das erfindungsgemäße Verfahren soll nun durch die nachfolgenden Beispiele erläutert werden. Dem Fachmann ist bewusst, dass die Beispiele nur zur Veranschaulichung dienen und als nicht limitierend anzusehen sind.

### BEISPIELE

### HERSTELLUNG DER KATALYSATOR-LÖSUNG

Eine 31 Glasapparatur mit Argon spülen und mit 21 Methanol befüllen. Das Methanol 1 Std. unter Rückfluss und Durchleiten von Argon kochen. Nach Abkühlen auf 20-25 °C, 7,2 g Di-Chloro-bis-[(Cycloocta-1,5-dien)rhodium (I), und 9,8 g (2R,4R)-4-(Dicyclohexylphosphino)-2-(diphenylphosphinomethyl)-N-methylaminocarbonyl-pyrrolidin unter Argon-Gegenstrom zugeben. Den Inhalt 30 Min. bei 20-25°C rühren. Dabei lösen sich die beiden Katalysatorkomponenten bis auf eine geringe Restmenge auf.

### HYDRIERUNG

90 kg Lobelanin Hydrochlorid in einen 300 1 Rührwerksapparat einfüllen und danach 174 kg Methanol einziehen. Anschließend den Inhalt 5 Min. rühren und die Suspension in einen inertisierten 500 Autoklaven einziehen. Über den 300 Rührwerksapparat eine Lösung aus 0,65 1 Triethylamin in 35,6 kg Methanol in den Autoklav einziehen. Den Rührer des Autoklaven an- und auf 400-500 U/Min. einstellen. Den Autoklav bis auf 700 mbar evakuieren und danach bis auf 1,5 bar mit Stickstoff begasen. Den Vorgang 10 mal wiederholen und danach den Apparat auf Normaldruck stellen.

Die Katalysator-Lösung in den Autoklav eindosieren und diesen dann mit Wasserstoff von 4 bar beaufschlagen. Den Überdruck in die Vakuumpumpe ablassen. Diesen Vorgang 4 mal wiederholen. Den Autoklaveninhalt auf 47-53°C erwärmen, den Wasserstoffdruck auf 20 bar, und den Rührer auf maximale Drehzahl einstellen. Bei 120% Wasserstoffaufnahme der Theorie (130,1 bar) die Hydrierung beenden. Den Autoklaveninhalt auf 20-25°C abkühlen. Danach den Autoklav evakuieren (700 mbar) und mit Stickstoff ausgleichen. Die Hydrierlösung in einen 5001 Rührwerksapparat überführen und das Methanol unter reduziertem Druck bei 50-60°C abdestillieren (gegen Ende der Destillation bei 45-50°C). Zum Rückstand 1001 Wasser, 501 Toluol und 1,21 Salzsäure, 32%ig zugeben. Den Rührer anstellen und ca. 20 Min. (bei 35-40°C, pH: 0,5-1,5) kräftig rühren lassen. Nach Abstellen des Rührers die Phasen absetzen lassen und die wässrige Phase abtrennen. Anschließend die wässrige Phase nochmals mit 30 l Toluol extrahieren. Die wässrige Phase in 5001 Rührwerksapparat einziehen, 1501 Toluol und 23,1 kg Natronlauge, 45%ig, zugeben. Den Rührer anstellen und ca. 20 Min. (bei 35-40°C, pH: 12-13) kräftig rühren lassen. Nach Abtrennen der wässrige Phase, zwei weitere Mal mit 401 und 25 l Toluol extrahieren. Die Toluol-Phasen in 5001 Rührwerksapparat vereinen, 50 l Wasser zugeben und gut durchmischen. Nach Abstellen des Rührers die Phasen absetzen lassen und untere wässrige Phase abtrennen. Anschließend die Toluolphase nochmals mit 50 1 Wasser ausrühren. Nach erneutem Abtrennen der Wasserphase die Toluol-Phase bei 50-60°C unter 65-80 mbar einengen. Das Ende der Destillation erfolgt bei 40°C bei 4 bis 10 mbar. Zum Rückstand 301 *iso*-Propanol einziehen und dieses mit restlichem Toluol bei 40-45°C im Vakuum wieder abdestillieren. Nach erneuter Zugabe von 120 1 *iso*-Propanol unter langsamen Rühren auf 17-23°C abkühlen und den Apparateinhalt bei dieser Temperatur 3 Tage rühren lassen. Anschließend auf -5 bis -10°C kühlen und weitere 2 Stunden rühren. Die Kristallsuspension danach über eine Zentrifuge abschleudern. Das Kristallisat mit 50 1 Toluol nachwaschen und bei 40-50°C im Vakuumtrockenschrank trocknen.
Ausbeute: 1-Lobelin, 22,2-28,6 kg; 29-35% d.Th.

## Patentansprüche

1. Verfahren zur Herstellung von L-Lobelin, der Formel **I**, 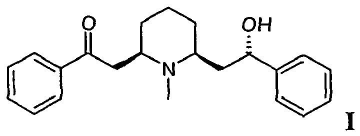 oder ein Säureadditionssalz davon, ausgehend von Lobelanin der Formel **II**, 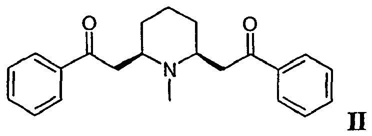 oder einem Säureadditionssalz davon, **dadurch gekennzeichnet, dass** man dieses einer asymmetrischen Hydrierung in Gegenwart eines Katalysatorsystems bestehend aus Rhodium und (2R,4R)-4-(Dicyclohexylphosphino)-2-(diphenylphosphino-methyl)-N-methyl-aminocarbonyl-pyrrolidin, gegebenenfalls einem inerten Verdünnungsmittels und einer schwachen Base unterwirft.

2. Verfahren nach Anspruch 1, worin man die Hydrierung in Gegenwart von weniger als einem Äquivalent einer schwachen Base ausgewählt aus der Gruppe der tertiären Amine durchgeführt wird.

3. Verfahren nach einem der Ansprüche 1 oder 2, worin die asymmetrische Hydrierung in einem Temperaturbereich von 0°C bis 100°C durchgeführt wird.

4. Verfahren nach einem der Ansprüche 1 bis 3, worin die asymmetrische Hydrierung unter einem Druck von 1 bis 40 bar durchgeführt wird.

5. Verfahren nach einem der Ansprüche 1 bis 5, worin die asymmetrische Hydrierung in einem protischen Verdünnungsmittel durchgeführt wird.

6. Verfahren nach Anspruch 5, worin die Hydrierung unter Schutzgas erfolgt.

7. Verfahren nach einem der Ansprüche 1 bis 6, worin man eine Verbindung der Formel **II** oder ein Säureadditionssalz davon zum Rhodiumkatalysator bei der asymmetrischen Hydrierung in einem Mol-Verhältnis von 500:1 bis 100000:1 einsetzt.

8. Verfahren nach einem der Ansprüche 1 bis 7, worin folgende Schritte durchgeführt werden:
• Lösen von Lobelanin in Methanol,
• Zugabe von Triethylamin in Methanol,
• Zugabe der Katalysator-Lösung,
• Beaufschlagung der Reaktionsmischung mit Wasserstoff ,
• Erhitzen der Reaktionsmischung auf 20-80°C und Einstellen des Wasserstoffdruck auf 10-30 bar,
• Nach Beendigung der Reaktion, Abdestillieren des Methanols unter reduziertem Druck.

9. Verfahren nach einem der Ansprüche 1 bis 8, worin zur Isolierung des Produktes folgende Schritte durchgeführt werden:
(i) Verteilung der bei der asymmetrischen Hydrierung erhaltenen Reaktionsmischung zwischen Wasser und einem organischen Lösungsmittel,
(ii) Einstellen eines pH-Wertes der wässrigen Phase im sauren Bereich,
(iii) Abtrennen der wässrigen Phase,
(iv) gegebenenfalls Wiederholung der Schritte (i) bis (iii)
(v) Einstellen des pH-Wertes der wässrigen Phase im basischen Bereich;
(vi) Verteilung der Reaktionsmischung zwischen Wasser und einem organischen Lösungsmittel,
(vii) gegebenenfalls Wiederholung der Schritte (v) bis (vi)
(viii) Abtrennen der gebildeten organischen Phase und Konzentrierung.
(ix) Kristallisieren des Produktes

10. Verfahren nach Anspruch 9, worin das organische Lösungsmittel Toluol ist.

11. Verfahren nach einem der Ansprüche 9 oder 10, worin das Produkt in Schritt (ix) durch Zugabe eines C₁₋₈-Alkohols kristallisiert wird.

## Claims

1. Process for preparing L-lobeline of formula **I**, 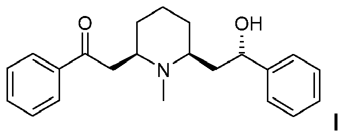 or an acid addition salt thereof, starting from lobelanine of formula **II**, 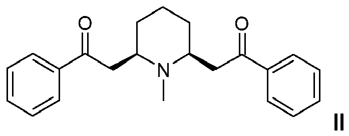 or an acid addition salt thereof, **characterised in that** the latter is subjected to asymmetric hydrogenation in the presence of a catalyst system consisting of rhodium and (2R,4R)-4-(dicyclohexylphosphino)-2-(diphenylphosphinomethyl)-N-methyl-aminocarbonyl-pyrrolidine, optionally an inert diluent and a weak base.

2. Process according to claim 1, wherein the hydrogenation is carried out in the presence of less than one equivalent of a weak base selected from among the tertiary amines.

3. Process according to one of claims 1 or 2, wherein the asymmetric hydrogenation is carried out in a temperature range from 0°C to 100°C.

4. Process according to one of claims 1 to 3, wherein the asymmetric hydrogenation is carried out at a pressure of 1 to 40 bar.

5. Process according to one of claims 1 to 5, wherein the asymmetric hydrogenation is carried out in a protic diluent.

6. Process according to claim 5, wherein the hydrogenation is carried out under protective gas.

7. Process according to one of claims 1 to 6, wherein a compound of formula **II** or an acid addition salt thereof is used in the asymmetric hydrogenation in a molar ratio of 500:1 to 100000:1 in relation to the rhodium catalyst.

8. Process according to one of claims 1 to 7, wherein the following steps are carried out:
• Dissolving the lobelanine in methanol,
• Adding triethylamine in methanol,
• Adding the catalyst solution,
• Treating the reaction mixture with hydrogen ,
• Heating the reaction mixture to 20-80°C and adjusting the hydrogen pressure to 10-30 bar,
• After the end of the reaction, distilling off the methanol under reduced pressure.

9. Process according to one of claims 1 to 8, wherein the following steps are carried out in order to isolate the product:
(i) distributing the reaction mixture obtained during asymmetric hydrogenation between water and an organic solvent,
(ii) adjusting the aqueous phase to a pH in the acidic range,
(iii) separating off the aqueous phase,
(iv) optionally repeating steps (i) to (iii)
(v) adjusting the aqueous phase to a pH in the basic range;
(vi) distributing the reaction mixture between water and an organic solvent,
(vii) optionally repeating steps (v) to (vi)
(viii) separating off the organic phase formed and concentrating it,
(ix) crystallising the product.

10. Process according to claim 9, wherein the organic solvent is toluene.

11. Process according to one of claims 9 or 10, wherein the product in step (ix) is crystallised by the addition of a C₁₋₈-alcohol.

## Revendications

1. Procédé de production de L-lobéline de formule I, 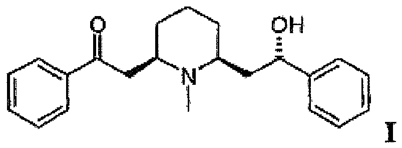 ou d'un sel d'addition acide de celle-ci, à partir de lobélanine de formule II, 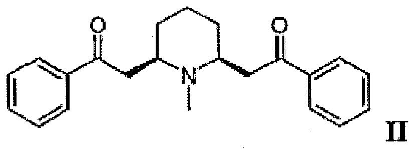 ou d'un sel d'addition acide de celle-ci, **caractérisé en ce que** l'on soumet celle-ci à une hydrogénation asymétrique en présence d'un système de catalyseur consistant en du rhodium et en de la (2R,4R)-4-(dicyclohexylphosphino)-2-(diphénylphosphinométhyl)-N-méthylaminocarbonyl-pyrrolidine, éventuellement un diluant inerte et une base faible.

2. Procédé selon la revendication 1, dans lequel l'hydrogénation est réalisée en présence de moins d'un équivalent d'une base choisie dans le groupe des amines tertiaires.

3. Procédé selon l'une des revendications 1 ou 2, dans lequel l'hydrogénation asymétrique est réalisée dans une plage de températures de 0°C à 100°C.

4. Procédé selon l'une des revendications 1 à 3, dans lequel l'hydrogénation asymétrique est réalisée à une pression de 1 à 40 bars.

5. Procédé selon l'une des revendications 1 à 5, dans lequel l'hydrogénation asymétrique est réalisée dans un diluant protique.

6. Procédé selon la revendication 5, dans lequel l'hydrogénation s'effectue sous un gaz protecteur.

7. Procédé selon l'une des revendications 1 à 6, dans lequel on ajuste un composé de formule II ou un sel d'addition acide de celui-ci au catalyseur rhodium lors de l'hydrogénation asymétrique en un rapport molaire de 500:1 à 100000:1.

8. Procédé selon l'une des revendications 1 à 7, dans lequel les étapes suivantes sont réalisée
- dissolution de la lobélanine dans du méthanol,
- addition de triéthylamine dans du méthanol,
- addition de la solution de catalyseur,
- apport d'hydrogène dans le mélange réactionnel
- chauffage du mélange réactionnel à 20-80°C et ajustement de la pression d'hydrogène à 10-30 bars,
- après la fin de la réaction, distillation du méthanol sous pression réduite.

9. Procédé selon l'une des revendications 1 à 8, dans lequel, pour isoler le produit, on réalise les étapes suivantes :
(i) répartition du mélange réactionnel obtenu par hydrogénation asymétrique entre l'eau et un solvant organique,
(ii) ajustement d'un pH de la phase aqueuse dans une plage acide,
(iii) séparation de la phase aqueuse,
(iv) éventuellement, répétition des étapes (i) à (iii)
(v) ajustement du pH de la phase aqueuse dans la plage basique ;
(vi) répartition du mélange réactionnel entre l'eau et un solvant organique,
(vii) éventuellement répétition des étapes (v) à (vi)
(viii) séparation de la phase organique formée et concentration
(ix) cristallisation du produit.

10. Procédé selon la revendication 9, dans lequel le solvant organique est le toluène.

11. Procédé selon l'une des revendications 9 ou 10, dans lequel le produit est cristallisé à l'étape (ix) par addition d'un alcool en C₁ à C₈.
